(19) Europäisches Patentamt
European Patent Office
Office européen des brevets

(11) EP 3 025 706 A1

(12) DEMANDE DE BREVET EUROPEEN

(43) Date de publication:
**01.06.2016 Bulletin 2016/22**

(21) Numéro de dépôt: **15191226.8**

(22) Date de dépôt: **18.08.2008**

(51) Int Cl.:
***A61K 9/20*** *(2006.01)* ***A61K 31/64*** *(2006.01)*
***A61P 3/10*** *(2006.01)*

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **21.03.2008 FR 0801561**

(62) Numéro(s) de document de la (des) demande(s) initiale(s) en application de l'article 76 CBE:
**08290778.3 / 2 103 302**

(27) Demande déposée antérieurement:
**18.08.2008 EP 08290778**

(71) Demandeur: **Les Laboratoires Servier**
**92284 Suresnes Cedex (FR)**

(72) Inventeurs:
- **Fonknechten, Gilles**
**45240 Ligny le Ribauld (FR)**
- **Genty, Patrick**
**45100 Orléans (FR)**
- **Péan, Jean-Manuel**
**45000 Orléans (FR)**
- **Wüthrich, Patrick**
**45560 Saint Denis en Val (FR)**

Remarques:
Cette demande a été déposée le 23-10-2015 comme demande divisionnaire de la demande mentionnée sous le code INID 62.

# (54) FORME GALÉNIQUE SÉCABLE PERMETTANT UNE LIBÉRATION MODIFIÉE DU PRINCIPE ACTIF

(57) Forme galénique sécable à libération modifiée de principe actif dont la forme galénique non subdivisée et une fraction de ladite forme obtenue par subdivision ont un profil de dissolution identique.
Médicaments.

EP 3 025 706 A1

## Description

La présente invention s'inscrit dans le cadre de la recherche et de la mise au point de nouvelles formes galéniques de préparations pharmaceutiques. La présente invention concerne une forme galénique sécable permettant une libération modifiée du principe actif.

Les compositions pharmaceutiques à libération modifiée, telles que les libérations prolongées, retardées ou séquentielles, de la substance active sont connues depuis longtemps. Elles permettent, en particulier, d'éviter des pics sanguins de principe actif et d'obtenir une concentration sanguine régulière chez l'homme. Ceci consiste à réduire les effets indésirables pouvant survenir par "effet de pic" éventuellement accompagné de troubles de type hydroélectrolytiques et métaboliques liés aux variations des taux plasmatiques du principe actif. Une forme à libération modifiée est particulièrement avantageuse par rapport à une forme à libération immédiate en évitant, chez certains sujets, l'obtention de concentrations sanguines de principe actif importantes et de courte durée dont l'effet peut s'avérer néfaste dans le traitement de certaines pathologies.

Une forme galénique sécable, tel qu'un comprimé sécable, a des caractéristiques, telles que des barres de cassures, permettant le fractionnement de cette forme galénique et aboutissant à des fractions de masses pratiquement égales contenant des quantités pratiquement égales de la substance active. La subdivision d'un comprimé constitue un problème classique et néanmoins récurrent en galénique. On trouve couramment des comprimés portant des rainures de ruptures permettant une cassure facile ainsi que l'obtention de doses de fractionnement contenant une quantité exacte et égale de principe actif.

La présente invention a pour but de conférer à une même forme galénique des propriétés classiques et néanmoins antagonistes que sont la qualité d'être sécable et la libération modifiée.

L'association des propriétés de sécabilité et de libération prolongée au sein d'une même forme galénique est explicitement déconseillée dans la directive CPMP/QWP/604/96 de l'EMEA, l'Agence Européenne du Médicament « C'est une mauvaise pratique de subdiviser les formes à libération prolongée mais cela pourrait être justifié dans des cas exceptionnels. ».

En effet, des comprimés présentant des rainures de rupture relativement profondes permettent une rupture plus facile desdits comprimés et un dosage exact de la substance active dans chaque dose fractionnaire. Cependant, ces comprimés sécables aux rainures de rupture profondes utilisés en doses fractionnaires présentent une augmentation importante de leurs surfaces pouvant atteindre 20% de la surface totale, correspondant à la surface de rupture. Cette augmentation significative de surface, dans le cas des doses fractionnaires, a pour effet de profondément perturber les caractéristiques de libération de la substance active. En conséquence, avec une surface totale considérablement agrandie par la subdivision, la libération modifiée de la substance active des doses fractionnaires est modifiée au point que lesdites doses fractionnaires ne possèdent plus ou ne possèdent plus qu'en partie les propriétés voulues en particulier une libération modifiée linéaire. En conséquence, l'utilisation de comprimés sécables présentant des rainures de rupture relativement profondes entraîne une incertitude de propriétés et d'efficacité qui n'est pas acceptable pour le patient.

Des solutions galéniques ont été envisagées afin de pallier aux problèmes liés à la subdivision des formes galéniques à libération modifiée.

En particulier, une forme originale de comprimés sécables à libération modifiée a été développée afin que l'augmentation de la surface totale due aux surfaces de rupture soit aussi faible que possible à la subdivision (FR 2 462 908). Ce comprimé de forme oblongue a des proportions relatives longueur/largeur/hauteur précises de 2.5 à 5 / 0.9 à 2 / 1. De plus, la largeur représente au maximum les 2/3 de la longueur et la profondeur totale des rainures est ajustée entre 1/3 à 1/2 de la hauteur de sorte que le produit d'une surface de rupture par le nombre des fragments possibles constitue au maximum 15% de la surface extérieure du comprimé non subdivisé. Cependant, en raison de la facilité accrue de rupture de ces comprimés sécables, compte-tenu de la faible surface des surfaces de rupture, lesdits comprimés ont tendance à la rupture au niveau de la barrette de sécabilité, phénomène délétère au cours du processus industriel.

La présente invention a donc pour but de proposer une stratégie alternative permettant de contourner les problèmes inhérents au développement de comprimés sécables à libération modifiée déjà disponibles, en vue de remédier, au moins en partie aux inconvénients liés à la subdivision des comprimés en dose fractionnaire. Cette stratégie alternative est fondée sur l'originalité de la composition pharmaceutique de la forme galénique.

La présente invention a pour objet une forme galénique sécable, par exemple un comprimé sécable, à libération modifiée comprenant un ou plusieurs principes actifs et les excipients suivants : un polymère dérivé de cellulose et un liant. Cette nouvelle forme galénique se caractérise par le fait qu'elle présente un profil de dissolution identique qu'elle ait été subdivisée ou non. Par exemple, le comprimé sécable à libération prolongée dans sa forme non subdivisée et une fraction de ladite forme obtenue par subdivision ont un profil de dissolution identique.

Dans le contexte de l'invention on entend par « profil de dissolution identique » des cinétiques de dissolution ayant des coefficients de variations sans différence statistiquement significative. Les cinétiques de dissolution in vitro identiques selon l'invention donnent des cinétiques plasmatiques identiques.

L'expression « principe actif » selon l'invention comprend le principe actif en tant que tel ou un de ses hydrates,

formes cristallines, et sels d'addition à un acide pharmaceutiquement acceptable.
Selon l'invention, l'expression « substance active » ou « principe actif » concerne, de manière non limitative, les familles thérapeutiques suivantes : antibiotiques, cardiovasculaires, analgésiques, anti-coagulants, anti-thrombotiques, vasoconstricteurs, vasodilatateurs, anti-tumoraux, hyper- et hypo-glycémiants, anti-inflammatoires, anti-arythmiques, anti-cholestérolémiants, vitamines, minéraux, chacun de ces principes actifs pouvant être associé entre eux.
De préférence, le principe actif selon l'invention est un hypoglycémiant, en particulier un antidiabétique. De préférence encore le principe actif est un dérivé de sulfonylurée.
De manière préférée, le principe actif utilisé dans l'invention est le gliclazide de formule (I) :

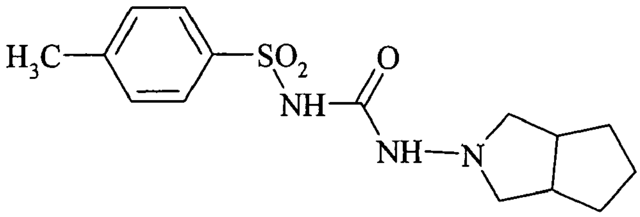

Le gliclazide est un dérivé de sulphonylurée reconnu pour ses propriétés antidiabétiques.
La posologie unitaire de gliclazide peut varier selon l'âge et le poids du patient, la nature et la sévérité du diabète. D'une façon générale, elle s'échelonne entre 30 et 120mg, en une seule prise, pour un traitement journalier. Le pourcentage de gliclazide, au sein d'une forme galénique, est compris entre 12 et 40% de la masse totale du comprimé.
Les formulations existantes jusqu'à présent consistaient en :

- un comprimé à libération immédiate dosé à 80mg ; et
- un comprimé matriciel dosé à 30mg de gliclazide. Ce comprimé permet de respecter la posologie unitaire qui s'échelonne entre 30 et 120 mg, en une seule prise journalière, correspondant à l'absorption de 1 à 4 comprimés dosés à 30 mg. Ce comprimé de gliclazide administré sous la forme d'une matrice hydrophile décrite dans le brevet EP 1 148 871 permet une libération prolongée et contrôlée du principe actif sans influence du pH sur la cinétique de dissolution *in vitro* de ladite matrice. Cette forme à libération prolongée de gliclazide permet d'assurer des taux plasmatiques réguliers ainsi que des variations $C_{max}$-$C_{min}$ faibles

Le schéma posologique recommandé pour le gliclazide consiste à administrer dans une première période du gliclazide à une dose de 30mg puis dans une deuxième période du gliclazide une dose de 60mg, dose de traitement administrée à la majorité des patients. Par ailleurs, des patients plus gravement atteints par la maladie doivent être traités à des doses de 90mg voire 120mg de gliclazide.
De manière très avantageuse par rapport aux formulations existantes, la présente invention consistant en un comprimé matriciel sécable à libération prolongée de gliclazide 60mg assure une meilleure observance du traitement en limitant le nombre de comprimés à prendre de la part du patient et permet également d'optimiser la fabrication des médicaments sur une unique ligne de production.
Dans la formule, le polymère dérivé de cellulose a pour fonction de former la matrice assurant, entre autre, la libération modifiée du principe actif. La libération du principe actif se fait à la fois par diffusion et par érosion de la matrice et permet en particulier une libération prolongée du principe actif.
Au sens de l'invention, les dérivés de celluloses, ou polymères cellulosiques, sont par exemple l'éthylcellulose, la méthylcellulose, l'acétate de cellulose, l'acétate phtalate de cellulose, l'hydroxyméthylcellulose, l'hydroxyéthylcellulose, l'hydroxypropylcellulose et l'hydroxypropylméthylcellulose.
Les dérivés de cellulose préférés selon l'invention sont : l'hydroxyméthylcellulose, l'hydroxyéthylcellulose, l'hydroxypropylcellulose et l'hydroxypropylméthylcellulose.
De préférence, le comprimé selon l'invention comprend un dérivé de cellulose de faible viscosité. De manière encore préférée, le comprimé selon l'invention comprend de l'hydroxypropylmethylcellulose ou HPMC.
Les HPMC sont des polymères disponibles dans le commerce, connus de l'homme du métier et habituellement utilisés dans le domaine de la formulation des médicaments. Notons que ces polymères sont notamment commercialisés sous la marque Methocel™ et Metolose™.
Une HPMC de viscosité élevée peut être choisie parmi Methocel K15M™ et Methocel K100M™, dont des solutions aqueuses à 2% en poids présentent des viscosités respectives de 15000 et 100000 cP.
Une HPMC de viscosité moyenne peut être choisie parmi Methocel E4M™, Methocel K4M™ et Methocel K4MCR™, dont des solutions aqueuses à 2 % en poids présentent une viscosité de 4000 cP.
Une HPMC de faible viscosité peut être choisie parmi Methocel E5™, Methocel E5 LV™, Methocel E15 LV™, Methocel E50 LV™, Methocel K100 LV™ et Metolose 90SH100™, dont des solutions aqueuses à 2 % en poids présentent des

viscosités respectives de 5, 5, 15, 50, 100 et 100 cP.

Dans la composition pharmaceutique selon l'invention le liant sert à agglutiner entre elles les particules qui ne peuvent l'être sous la seule action de la pression.

L'invention porte, de préférence, sur les liants compris dans la liste suivante : solution de saccharose, de glucose ou de sorbitol, sirop de glucose, de préférence la maltodextrine, gomme arabique, adragante, méthylcellulose, carboxyméthylcellulose, gélatine, amidons, PEG 4000 et 6000, polyvidone (PVP) et HPMC de très faible viscosité.

De manière préférée, le comprimé selon l'invention comprend de la maltodextrine, de la polyvidone ou une HPMC de très faible poids moléculaire en tant que liant de la présente forme galénique.

La présente invention concerne donc de préférence un comprimé sécable à libération prolongée comprenant : a) du gliclazide, un dérivé de cellulose, de la maltodextrine ou b) du gliclazide, un dérivé de cellulose, de la polyvidone ou c) du gliclazide, un dérivé de cellulose, une HPMC de faible à très faible poids moléculaire.

Dans un mode de réalisation préféré, le comprimé selon l'invention comprend également un hydrophylisant. Conformément à l'acception usuelle, on entend par hydrophylisant toute substance capable de faciliter la pénétration d'eau dans la matrice afin de rapidement former un gel. Dans le contexte de l'invention les hydrophylisants sont compris dans la liste suivante : silice colloïdale, polysorbate, ester de sorbitol. Avantageusement, le comprimé selon l'invention comprend de la silice colloïdale en tant qu'hydrophylisant de la présente forme galénique. Le pourcentage de silice colloïdale comme hydrophylisant dans le comprimé selon l'invention est compris entre 0.1% et 5% de la masse totale du comprimé.

La présente invention porte en particulier sur un comprimé sécable à libération prolongée comprenant du gliclazide, un dérivé de cellulose, de la maltodextrine et de la silice colloïdale.

La présente invention porte également sur un comprimé comprenant, outre les principes actifs et les excipients déjà décrits,

- au moins un diluant ou agent de charge tel que le lactose monohydraté, manitol, polyol, cellulose non substituée ou également l'amidon et un sel minéral, phosphate dicalcique ; et/ou
- au moins un lubrifiant en particulier un lubrifiant de compression tel que le stéarate de magnésium ou également le stéarate de calcium, de zinc et d'aluminium, le stéaryl fumarate de sodium ; et/ou
- au moins un agent de coulance tel que la silice colloïdale anhydre.

De préférence, l'invention concerne un comprimé sécable à libération modifiée comprenant entre 12% et 40% de principe actif de la masse totale du comprimé. De manière préférée, le comprimé sécable selon l'invention comprend également entre 10% et 60% de dérivés de cellulose de la masse totale du comprimé. Tout particulièrement, le comprimé sécable selon l'invention comprend entre 2% et 15% de liant de la masse totale du comprimé.

Par ailleurs, le comprimé sécable selon l'invention porte une ou plusieurs rainures de rupture disposées sur une face ou sur les deux, perpendiculairement aux directions de la hauteur et de la longueur du comprimé. Les rainures de rupture pratiquées sur les deux faces sont de préférence en face les unes des autres ou encore alternées, et en outre de profondeur identique ou différente. Le comprimé sécable peut donc être fragmenté en deux ou plusieurs parties pré-déterminées. Il en résulte une possibilité de dosage du médicament adaptée à la posologie spécifique liée à la pathologie ou au patient.

L'invention porte de manière préférée sur un comprimé sécable tel que 13 à 27% de la quantité totale de substance active est libérée après 2 heures, 32 à 52% de la quantité totale de substance active est libérée après 4 heures et que plus de 85% de la quantité totale de substance active est libérée après 12 heures.

De manière préférée, le comprimé selon l'invention présente la formule unitaire (en mg/comprimé) et la formule en pourcentage suivantes :

<u>L0014022 :</u>

| | | |
|---|---|---|
| - gliclazide | 60,00 | 18.7% |
| | | |
| - lactose monohydraté | 71,36 | 22.3% |
| - HPMC 100cP | 160,00 | 50% |
| - maltodextrine | 22,00 | 6.9% |
| - silice colloïdale anhydre | 5,04 | 1.6% |
| - stéarate de magnésium | 1,60 | 0.5% |
| | | |
| Masse totale : | 320,00 | |

La formulation suivante du comprimé selon l'invention est donnée en fonction d'une part, de la quantité en mg/masse totale de chaque composé et d'autre part, de la position en phase interne ou externe dudit composé :

<u>L0014022 :</u>

| | |
|---|---|
| Phase interne : | |
| Gliclazide | 60 |
| Lactose | 71.36 |
| HPMC 100cP | 64 |
| Maltodextrin | 22 |
| Silice colloïdale anhydre | 4.4 |
| | |
| Phase externe : | |
| HPMC 100cP | 96 |
| Stéarate de magnésium | 1.6 |
| Silice colloïdale anhydre | 0.64 |
| | |
| Masse totale | 320 |

L'invention s'étend au procédé de préparation par granulation humide d'un comprimé sécable tel que décrit supra, comprenant au moins les étapes suivantes :

a) mélange de gliclazide, de maltodextrine, de lactose monohydraté, d'une partie du dérivé de cellulose et d'une partie de la silice colloïdale ;
b) après mélange réaliser le mouillage. La masse humide ainsi obtenue étant ensuite granulée, séchée puis calibrée ;
c) le granulé obtenu à l'étape b) constitue une phase interne et est mélangé à la partie restante du dérivé de cellulose de faible viscosité ;
d) lubrification du granulé obtenu à l'étape c) au moyen de silice colloïdale et de stéarate de magnésium ;
e) compression du mélange lubrifié en utilisant des poinçons permettant la réalisation des rainures de rupture sur le comprimé.

L'invention s'étend également au procédé de préparation par compression directe d'un comprimé sécable tel que décrit supra, comprenant au moins les étapes suivantes :

a) mélange de gliclazide, de maltodextrine, de lactose monohydraté, de dérivés de cellulose et d'une partie de la silice colloïdale ;
b) lubrification du mélange obtenu à l'étape a) au moyen de silice colloïdale et de stéarate de magnésium ;
c) compression du mélange lubrifié en utilisant des poinçons permettant la réalisation des rainures de rupture sur le comprimé.

L'invention s'étend enfin au procédé de préparation par granulation par compactage ou par granulation sèche d'un comprimé sécable tel que décrit supra, comprenant au moins les étapes suivantes :

a) mélange de gliclazide, de maltodextrine, de lactose monohydraté, d'une partie du dérivé de cellulose et d'une partie de la silice colloïdale ;
b) après mélange réaliser le compactage puis calibrée ;
c) le granulé obtenu à l'étape b) constitue une phase interne et est mélangé à la partie restante du dérivé de cellulose de faible viscosité ;
d) lubrification du granulé obtenu à l'étape c) au moyen de silice colloïdale et de stéarate de magnésium ;
e) compression du mélange lubrifié en utilisant des poinçons permettant la réalisation des rainures de rupture sur le comprimé.

De préférence, on obtient en fin de procédé des comprimés dont la dureté mesurée par écrasement diamétral va d'environ 60 à 120 N et dont le fractionnement à l'aide des rainures de rupture facilite l'observance du traitement.
De préférence les formes galéniques sécables à libération modifiée de gliclazide selon l'invention sont utilisées dans la fabrication de médicaments pour le traitement du diabète.
La présente invention est illustrée sans pour autant être limitée par les figures et exemples suivants :

Figure 1 : Cinétique de dissolution comparée d'un comprimé entier et d'un demi comprimé de composition L0014022 ;
Figure 2 : Cinétique de dissolution comparée d'un comprimé entier et d'un demi comprimé de composition L0023844 ;
Figure 3 : Cinétique de dissolution comparée d'un comprimé entier et d'un demi comprimé de composition L0023845 ;
Figure 4 : Cinétique de dissolution comparée d'un comprimé entier et d'un demi comprimé de composition L0023849.

**Exemples 1 à 4 : Comparaison des cinétiques de dissolution**

Les exemples 1 à 4 comparent la cinétique de libération *in vitro* de comprimés non-subdivisés à la cinétique de libération *in vitro* de doses fractionnaires. Les profils de dissolution sont comparés à l'aide du facteur de similarité ($f_2$). Deux profils de dissolution sont considérés comme similaires lorsque la valeur ($f_2$) est supérieure ou égale à 50. Le calcul du facteur de similarité ($f_2$) est conseillé par les directives de l'EMEA et de la FDA afin de comparer deux profils de dissolution et permettre de conclure à l'identité desdits profils de dissolution.

Le facteur de similarité ($f_2$) a pour formule :

$$f_2 = 50 \bullet \log \left( \frac{100}{\sqrt{1 + \frac{\sum_{t=1}^{t=n} \left[ \overline{R}(t) - \overline{T}(t) \right]^2}{n}}} \right)$$

dans laquelle $f_2$ est le facteur de similarité, n est le nombre de points normés, R(t) est la moyenne en pourcentage de principe actif dissout du comprimé non-subdivisé et T(t) est la moyenne en pourcentage de principe actif dissout d'une dose fractionnaire dudit comprimé. Dans les exemples 1 à 4 les points normés sont à t=2 heures, t=4 heures et t=12 heures.

Les comprimés évalués ont différentes formulations, ces formulations varient en particulier dans la nature des dérivés cellulosiques et la nature des liants utilisés. Les comprimés sont fabriqués selon le procédé de l'invention décrit supra.

Exemple 1 :

| L0014022 | Quantité mg |
|---|---|
| Gliclazide | 60 |
| HPMC 100cP | 64 |
| HPMC 100cP (phase externe) | 96 |
| Povidone | 22 |
| Lactose Monohydrate | 71,36 |
| Silice Colloidale Anhydre | 0,64 |
| Silice Colloidale Anhydre (phase externe) | 4,4 |
| Magnésium Stearate (phase externe) | 1,6 |

| Temps (heure) | L0014022 | L0014022 | L0014022 | L0014022 | L0014022 |
|---|---|---|---|---|---|
| | ½ comprimé | ½ comprimé | Comprimé entier | Comprimé entier | Comprimé entier / ½ comprimé |
| | % de PA libéré | Ecart type | % de PA libéré | Ecart type | $f_2$ |
| 0 | 0 | 0 | 0 | 0 | 75% |
| 0.5 | 4.34 | 0.33 | 3.61 | 0.95 | |
| 1 | 9.93 | 0.57 | 8.68 | 1.05 | |
| 2 | 22.38 | 1.07 | 20.25 | 2.09 | |
| 4 | 47.8 | 1.78 | 43.77 | 3.54 | |

(suite)

| Temps (heure) | L0014022 | L0014022 | L0014022 | L0014022 | L0014022 |
|---|---|---|---|---|---|
| | ½ comprimé | ½ comprimé | Comprimé entier | Comprimé entier | Comprimé entier / ½ comprimé |
| | % de PA libéré | Ecart type | % de PA libéré | Ecart type | $f_2$ |
| 8 | 90.17 | 2.87 | 84.64 | 3.26 | |
| 12 | 98.58 | 2.93 | 99.1 | 0.8 | |

Exemple 2 :

| L0023844 | mg |
|---|---|
| Gliclazide | 60 |
| HPMC 100cP | 64 |
| HPMC 100cP (phase externe) | 96 |
| Povidone | 22 |
| Lactose Monohydrate | 71,36 |
| Silice Colloidale Anhydre | 0,64 |
| Silice Colloidale Anhydre (phase externe) | 4,4 |
| Magnésium Stearate (phase externe) | 1,6 |

| Temps (heure) | L0023844 | L0023844 | L0023844 | L0023844 | L0023844 |
|---|---|---|---|---|---|
| | ½ comprimé | ½ comprimé | Comprimé entier | Comprimé entier | Comprimé entier / ½ comprimé |
| | % de PA libéré | Ecart type | % de PA libéré | Ecart type | $f_2$ |
| 0 | 0.00 | 0.00 | 0.00 | 0.00 | 52.8% |
| 0.5 | 3.17 | 0.24 | 2.79 | 0.48 | |
| 1 | 8.94 | 0.44 | 7.04 | 0.59 | |
| 2 | 22.11 | 1.04 | 17.65 | 1.19 | |
| 4 | 51.10 | 1.36 | 41.04 | 1.63 | |
| 8 | 102.52 | 2.42 | 84.31 | 1.52 | |
| 12 | 107.17 | 2.32 | 106.48 | 1.91 | |

Exemple 3 :

| L0023845 | mg |
|---|---|
| Gliclazide | 60 |
| HPMC 100cP | 64 |
| HPMC 100cP (phase externe) | 96 |
| Lactose Monohydrate | 71,36 |
| Silice Coiioidaie Anhydre | 0,64 |
| Silice Colloïdale Anhydre (phase externe) | 4,4 |
| Magnésium Stearate (phase externe) | 1,6 |
| HPMC faible poids moléculaire | 22 |

| Temps (heure) | L0023845 | L0023845 | L0023845 | L0023845 | L0023845 |
|---|---|---|---|---|---|
| | ½ comprimé | ½ comprimé | Comprimé entier | Comprimé entier | Comprimé entier / ½ comprimé |
| | % de PA libéré | Ecart type | % de PA libéré | Ecart type | $f_2$ |
| 0 | 0.00 | 0.00 | 0.00 | 0.00 | 62.2% |
| 0.5 | 3.53 | 0.68 | 2.97 | 0.05 | |
| 1 | 8.06 | 0.71 | 6.86 | 0.12 | |
| 2 | 19.15 | 0.88 | 15.87 | 0.61 | |
| 4 | 43.17 | 0.94 | 35.74 | 1.14 | |
| 8 | 84.60 | 1.58 | 73.65 | 1.49 | |
| 12 | 98.11 | 2.75 | 97.37 | 2.03 | |

Exemple 4 :

| L0023849 | mg |
|---|---|
| Maltodextrine | 22 |
| Hydroxyethycellulose | 64 |
| Hydroxyethycellulose (phase externe) | 96 |
| Gliclazide | 60 |
| Lactose Monohydrate | 71,36 |
| Silice Colloïdale Anhydre | 0,64 |
| Silice Colloïdale Anhydre (phase externe) | 4,4 |
| Magnésium Stearate (phase externe) | 1,6 |

| Temps (heure) | L0023849 | L0023849 | L0023849 | L0023849 | L0023849 |
|---|---|---|---|---|---|
| | ½ comprimé | ½ comprimé | Comprimé entier | Comprimé entier | Comprimé entier / ½ comprimé |
| | % de PA libéré | Ecart type | % de PA libéré | Ecart type | $f_2$ |
| 0 | 0.00 | 0.00 | 0.00 | 0.00 | 53.4% |
| 0.5 | 4.63 | 0.29 | 3.80 | 0.10 | |
| 1 | 8.91 | 0.31 | 7.27 | 0.25 | |
| 2 | 19.32 | 0.62 | 15.78 | 0.58 | |
| 4 | 41.34 | 1.04 | 37.47 | 2.02 | |
| 8 | 86.61 | 2.62 | 67.37 | 3.10 | |
| 12 | 95.71 | 2.76 | 95.00 | 1.92 | |

Les compositions pharmaceutiques évaluées dans les exemples 1 à 5 ont des profils de dissolution similaires pour la forme non subdivisée et pour la fraction de la forme obtenue par subdivision.

## Revendications

1. Comprimé sécable à libération modifiée comprenant un principe actif, un dérivé de cellulose et un liant tel que le comprimé non subdivisé et une fraction dudit comprimé obtenu par subdivision ont un profil de dissolution similaire.

2. Comprimé selon la revendication 1, **caractérisé en ce que** la substance active est le gliclazide.

**3.** Comprimé selon l'une des revendications 1 ou 2, **caractérisé en ce que** le dérivé de cellulose est l'hydroxyméthylcellulose, l'hydroxyéthylcellulose, l'hydroxypropylcellulose et/ou l'hydroxypropylméthylcellulose.

**4.** Comprimé selon l'une des revendications 1 à 3, **caractérisé en ce que** le dérivé de cellulose est l'hydroxypropylmethylcellulose de faible viscosité.

**5.** Comprimé selon l'une des revendications 1 à 4, **caractérisé en ce que** le liant est la maltodextrine, la polyvidone ou une hydroxypropylmethylcellulose de très faible viscosité.

**6.** Comprimé selon l'une des revendications 1 à 5, **caractérisé en ce que** le comprimé comprend un hydrophylisant, de préférence l'hydrophylisant est la silice colloïdale.

**7.** Comprimé selon l'une des revendications 1 à 6, **caractérisé en ce qu'**il comprend du gliclazide, un dérivé de cellulose, de la maltodextrine et de la silice colloïdale anhydre.

**8.** Comprimé selon l'une des revendications 1 à 7, **caractérisé en ce que** le pourcentage de substance active est compris entre 12% et 40% de la masse totale du comprimé.

**9.** Comprimé selon l'une des revendications 1 à 8, **caractérisé en ce que** le pourcentage de dérivé de cellulose est compris entre 10% et 60% de la masse totale du comprimé.

**10.** Comprimé selon l'une des revendications 1 à 9, **caractérisé en ce que** le pourcentage de liant est compris entre 2% et 15% de la masse totale du comprimé.

**11.** Comprimé selon l'une des revendications 2 à 10, **caractérisé en ce qu'**il comprend une quantité totale de gliclazide de 60mg.

**12.** Comprimé sécable à libération modifiée **caractérisé en ce qu'**il comprend 18.7% de gliclazide, 22.3% de lactose monohydrate, 6.9% de maltodextrine, 50% d'hydroxypropylmethylcellulose faiblement substituée, 0.5% de stéarate de magnésium et 1.6% de silice colloïdale anhydre.

**13.** Comprimé selon l'une des revendications 1 à 12, **caractérisé en ce qu'**il porte une ou plusieurs rainures de ruptures perpendiculaires à la hauteur et à la longueur du comprimé.

**14.** Comprimé selon l'une des revendications 1 à 13, **caractérisé en ce qu'**il permet une libération prolongée.

**15.** Comprimé selon l'une des revendications 1 à 14, **caractérisée en ce que** 13 à 27% de la quantité totale de substance active est libérée après 2 heures, 32 à 52% de la quantité totale de substance active est libérée après 4 heures et que plus de 85% de la quantité totale de substance active est libérée après 12 heures.

**16.** Comprimé selon l'une des revendications 1 à 15 utilisé dans la fabrication de médicament pour le traitement du diabète.

**17.** Procédé de fabrication d'un comprimé selon l'une des revendications 1 à 16, **caractérisé en ce qu'**il s'agit d'un procédé par granulation humide, granulation par compactage ou compression directe.

Figure 1

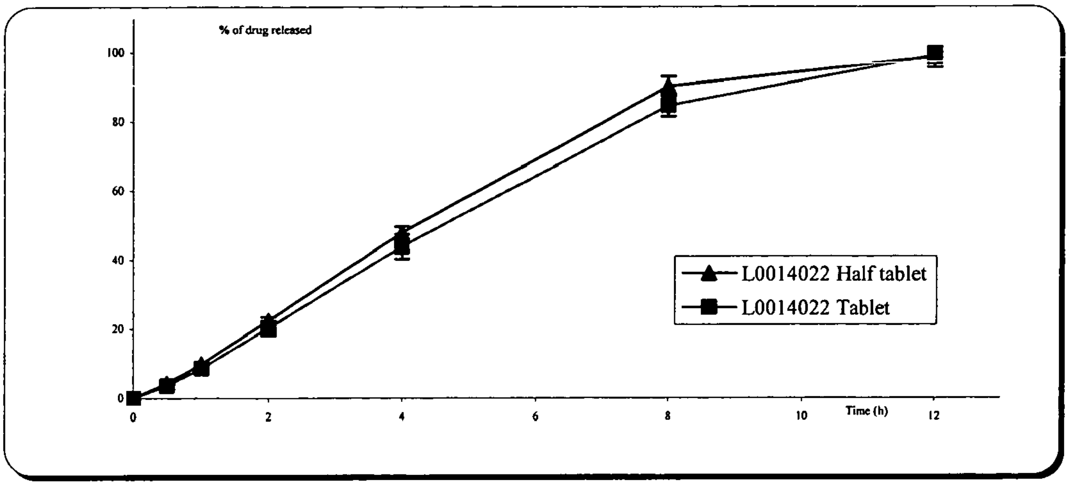
% of drug released
100
80
60
40
20
0
0
2
4
6
8
10
Time (h)
12
L0014022 Half tablet
L0014022 Tablet

Figure 2

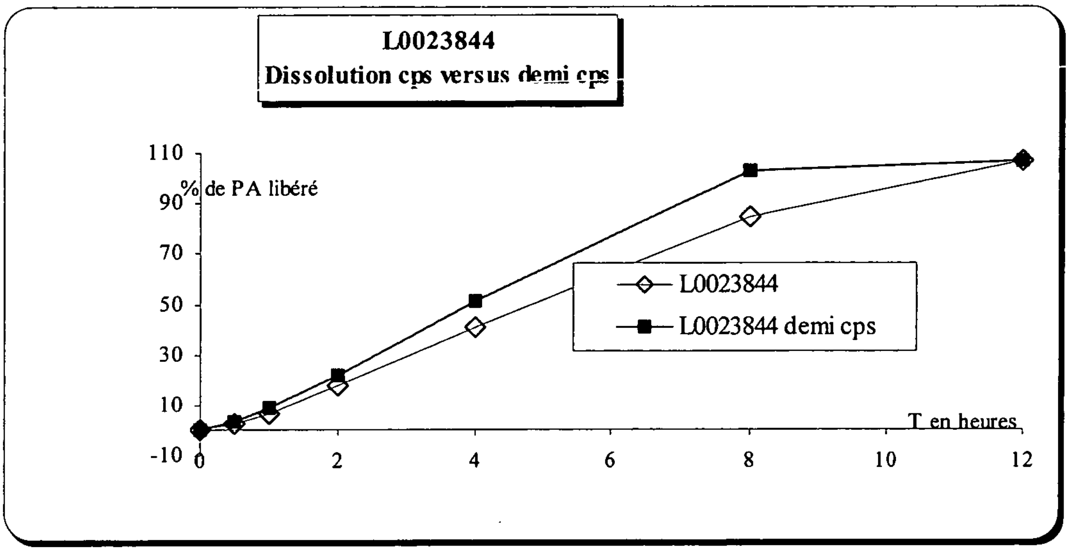
L0023844
Dissolution cps versus demi cps
110
% de PA libéré
90
70
50
30
10
-10
0
2
4
6
8
10
12
T en heures
L0023844
L0023844 demi cps

Figure 3

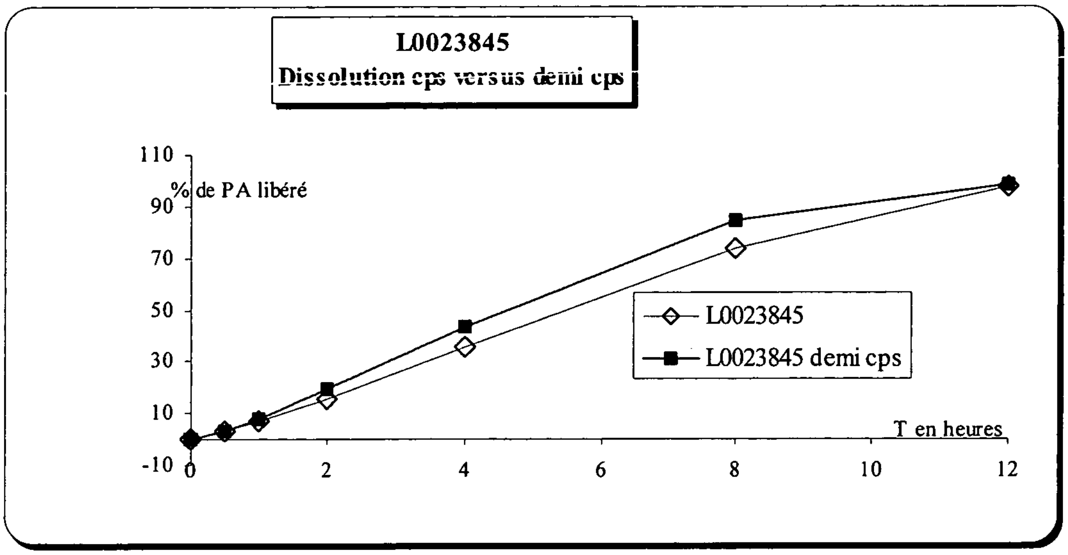
L0023845
Dissolution cps versus demi cps
% de PA libéré
110
90
70
50
30
10
-10
0
2
4
6
8
10
12
T en heures
L0023845
L0023845 demi cps

Figure 4

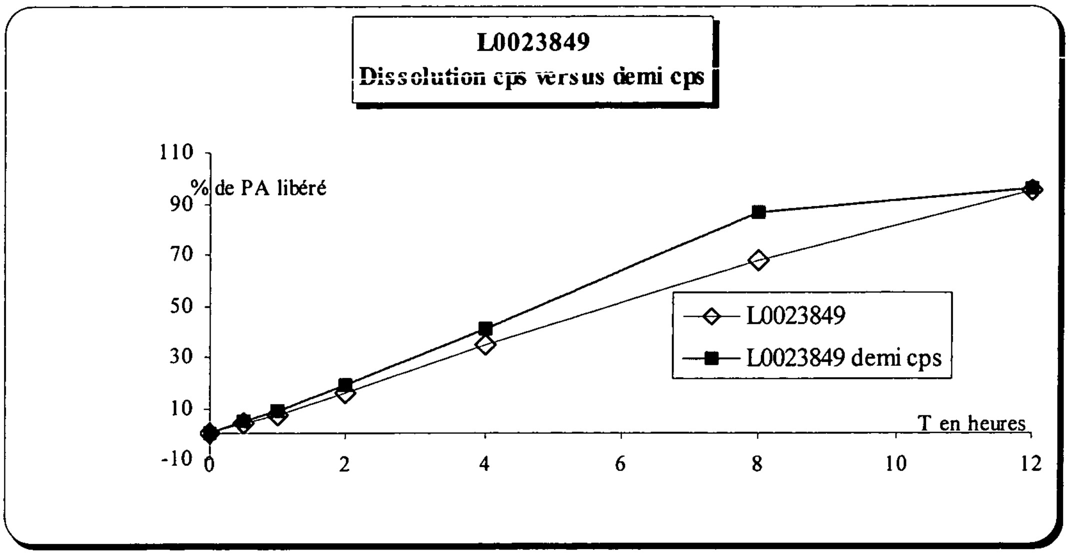
L0023849
Dissolution cps versus demi cps
110
% de PA libéré
90
70
50
30
10
-10
0
2
4
6
8
10
12
T en heures
L0023849
L0023849 demi cps

Europäisches Patentamt
European Patent Office
Office européen des brevets

# RAPPORT DE RECHERCHE EUROPEENNE

Numéro de la demande
EP 15 19 1226

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 462 908 A (CIBA GEIGY AG [CH]) 20 février 1981 (1981-02-20)<br>* figures *<br>* exemples 1,2 *<br>* page 2, ligne 17 - page 3, ligne 8 * | 1,3-6, 8-10, 13-15,17 | INV.<br>A61K9/20<br>A61K31/64<br>A61P3/10 |
| X | WO 00/18373 A (ADIR [FR]; HUET DE BAROCHEZ BRUNO [FR]; WUTHRICH PATRICK [FR]; MARTIN) 6 avril 2000 (2000-04-06)<br>* exemples * | 1-17 | |
| X | EP 1 741 435 A (LOTUS PHARMACEUTICAL CO LTD [TW]) 10 janvier 2007 (2007-01-10)<br>* exemples * | 1-17 | |
| X | WO 2006/061697 A (THEMIS LAB PRIVATE LTD [IN]; ANTARKAR AMIT KRISHNA [IN]; KAMDAR NIRAV) 15 juin 2006 (2006-06-15)<br>* exemples * | 1-17 | DOMAINES TECHNIQUES RECHERCHES (IPC) |
| A | DUMAN E ET AL: "Effect of scoring design on the uniformity of extended release matrix tablet halves", PHARMAZEUTISCHE INDUSTRIE, AULENDORF, DE, vol. 62, no. 7, 1 janvier 2000 (2000-01-01), pages 547-550, XP003029648, ISSN: 0031-711X<br>* page 547 *<br>* page 550, lignes 1-5 * | 13 | A61K |

Le présent rapport a été établi pour toutes les revendications

1

| Lieu de la recherche | Date d'achèvement de la recherche | Examinateur |
|---|---|---|
| La Haye | 3 février 2016 | Opravz, Petra |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 15 19 1226

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-02-2016

| Document brevet cité au rapport de recherche | Date de publication | Membre(s) de la famille de brevet(s) | Date de publication |
|---|---|---|---|
| FR 2462908 A | 20-02-1981 | AU 531058 B2 | 11-08-1983 |
| | | AU 6148180 A | 19-02-1981 |
| | | CA 1158555 A | 13-12-1983 |
| | | DE 3030622 A1 | 26-03-1981 |
| | | DK 354580 A | 17-02-1981 |
| | | ES 8106231 A1 | 16-10-1981 |
| | | FI 802542 A | 17-02-1981 |
| | | FR 2462908 A1 | 20-02-1981 |
| | | GB 2057878 A | 08-04-1981 |
| | | GR 69943 B | 21-07-1982 |
| | | IE 50108 B1 | 19-02-1986 |
| | | IL 60839 A | 30-11-1983 |
| | | IT 1145285 B | 05-11-1986 |
| | | LU 82718 A1 | 24-03-1981 |
| | | MY 8700198 A | 31-12-1987 |
| | | NL 8004616 A | 18-02-1981 |
| | | NO 802447 A | 17-02-1981 |
| | | NZ 194681 A | 17-06-1983 |
| | | PT 71706 A | 01-09-1980 |
| | | SE 445802 B | 21-07-1986 |
| | | US 4353887 A | 12-10-1982 |
| WO 0018373 A | 06-04-2000 | AP 1243 A | 02-02-2004 |
| | | AT 296621 T | 15-06-2005 |
| | | AU 764516 B2 | 21-08-2003 |
| | | AU 6098299 A | 17-04-2000 |
| | | BR 9917012 A | 16-04-2002 |
| | | CA 2273420 A1 | 01-08-2000 |
| | | CN 1342068 A | 27-03-2002 |
| | | CZ 20012661 A3 | 14-11-2001 |
| | | DE 69925639 D1 | 07-07-2005 |
| | | DE 69925639 T2 | 27-04-2006 |
| | | DK 1148871 T3 | 19-09-2005 |
| | | EE 200100398 A | 15-10-2002 |
| | | EP 1148871 A1 | 31-10-2001 |
| | | ES 2241323 T3 | 16-10-2005 |
| | | FR 2788981 A1 | 04-08-2000 |
| | | GE P20053501 B | 10-05-2005 |
| | | HK 1043049 A1 | 04-02-2005 |
| | | HR P20010632 A2 | 28-02-2005 |
| | | HU 0105365 A2 | 29-04-2002 |
| | | ID 30225 A | 15-11-2001 |
| | | IL 144246 A | 10-12-2006 |
| | | JP 4716465 B2 | 06-07-2011 |
| | | JP 2002525310 A | 13-08-2002 |
| | | JP 2008179649 A | 07-08-2008 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 15 19 1226

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-02-2016

| Document brevet cité au rapport de recherche | Date de publication | Membre(s) de la famille de brevet(s) | Date de publication |
|---|---|---|---|
| | | NO 20013757 A | 09-08-2001 |
| | | NZ 512878 A | 31-05-2002 |
| | | OA 11756 A | 19-07-2005 |
| | | PL 356707 A1 | 28-06-2004 |
| | | PT 1148871 E | 31-08-2005 |
| | | SK 10952001 A3 | 03-12-2001 |
| | | TR 200102002 T2 | 21-01-2002 |
| | | UA 68414 C2 | 17-12-2001 |
| | | US 6733782 B1 | 11-05-2004 |
| | | WO 0018373 A1 | 06-04-2000 |
| | | YU 53101 A | 12-05-2004 |
| | | ZA 200106305 A | 31-07-2002 |
| EP 1741435 A | 10-01-2007 | AU 2004318976 A1 | 10-11-2005 |
| | | CA 2563325 A1 | 10-11-2005 |
| | | EP 1741435 A1 | 10-01-2007 |
| | | JP 2007534704 A | 29-11-2007 |
| | | WO 2005105109 A1 | 10-11-2005 |
| WO 2006061697 A | 15-06-2006 | EP 1827453 A1 | 05-09-2007 |
| | | WO 2006061697 A1 | 15-06-2006 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2462908
- EP 1148871 A